# EUROPÄISCHE PATENTSCHRIFT

(11) **EP 1 472 210 B1**
(45) Veröffentlichungstag und Bekanntmachung des Hinweises auf die Patenterteilung: **26.11.2014**
(21) Anmeldenummer: 03704380.9
(22) Anmeldetag: 11.01.2003
(51) Int. Cl.: C07C 67/343, C07C 69/608, C07C 69/533, C07C 227/22, C07C 229/28, C07C 229/08

(54) **VERFAHREN ZUR HERSTELLUNG SUBSTITUIERTER ACRYLSÄUREESTER UND DEREN EINSATZ ZUR HERSTELLUNG VON SUBSTITUIERTEN GAMMA-AMINOSÄUREN**
METHODS FOR PRODUCING SUBSTITUTED ACRYLIC ACID ESTERS AND USE OF THE LATTER FOR PRODUCING SUBSTITUTED $G(G)-AMINO ACIDS
PROCEDES POUR LA PRODUCTION D'ACRYLATES SUBSTITUES ET LEUR UTILISATION POUR LA PRODUCTION DE GAMMA-AMINOACIDES SUBSTITUES

(30) Priorität: 25.01.2002 DE 10203122
(43) Veröffentlichungstag der Anmeldung: 03.11.2004
(73) Patentinhaber: Grünenthal GmbH, 52078 Aachen (DE)
(72) Erfinder: PRZEWOSNY, Michael, Thomas, 52064 Aachen (DE); PÜTZ, Claudia, 52349 Düren (DE)
(74) Vertreter: Kutzenberger, Helga
(86) Internationale Anmeldenummer: PCT/EP2003/000213
(87) Internationale Veröffentlichungsnummer: WO 2003/062185

(56) Entgegenhaltungen:
- J. VILLIERAS, M. RAMBAUD: "Wittig-Horner Reactions in Heterogeneous Media" SYNTHESIS., Bd. 4, 1983, Seiten 300-303, XP002239328 GEORG THIEME VERLAG. STUTTGART., DE ISSN: 0039-7881
- A. A. VASIL'EV, E. P. SEREBRYAKOV ET AL.: "A Versatile and Convinient Protocol for the Stereocontrolled Synthesis of Olefinic Insect Pheromones" BIOORGANIC & MEDICINAL CHEMISTRY., Bd. 4, Nr. 3, 1996, Seiten 389-400, XP002239329 ELSEVIER SCIENCE LTD., GB ISSN: 0968-0896
- F. BARBOT ET AL.: "Addition en 1,8 d'organocuprates lithiens saturés sur la cétone CH3(CH=CH)COCH3 et sur l'ester CH3(CH=CH)COOC2H5" JOURNAL OF ORGANOMETALLIC CHEMISTRY., Bd. 345, 1988, Seiten 239-243, XP002239330 ELSEVIER-SEQUOIA S.A. LAUSANNE., CH ISSN: 0022-328X
- I. GOMEZ-MONTERREY, B. P. ROQUES ET AL.: "Exploration of Neutral Endopeptidase Active Site by a Series of New Thiol-Containing Inhibitors" JOURNAL OF MEDICINAL CHEMISTRY., Bd. 36, 1993, Seiten 87-94, XP002239331 AMERICAN CHEMICAL SOCIETY. WASHINGTON., US ISSN: 0022-2623
- BRYANS J S ET AL: "IDENTIFICATION OF NOVEL LIGANDS FOR THE GABAPENTIN BINDING SITE ON THE ALPHA2DELTA SUBUNIT OF A CALCIUM CHANNEL AND THEIR EVALUATION AS ANTICONVULSANT AGENTS" JOURNAL OF MEDICINAL CHEMISTRY, AMERICAN CHEMICAL SOCIETY. WASHINGTON, US, Bd. 41, Nr. 11, 1998, Seiten 1838-1845, XP000941901 ISSN: 0022-2623 in der Anmeldung erwähnt

## Beschreibung

Die vorliegende Erfindung betrifft Verfahren zur Herstellung von substituierten Acrylsäureestern bzw. deren Einsatz zur Herstellung von substituierten γ-Aminosäuren, wie Gabapentin und Pregabalin.

Substituierte γ-Aminosäuren, wie Gabapentin der nachstehenden Formel (A) und Pregabalin der nachstehenden Formel (B), werden als Medikamente zur Behandlung von Epilepsie und Schmerzzuständen verwendet (J.S. Bryans, D.J. Wustrow; Medicinal Research Reviews 19, 149 - 177 (1999) und L. Martin, X. Rabasseda, P. Leeson, J. Castaner; Drugs of the Future 24, 862 - 870 (1999)).

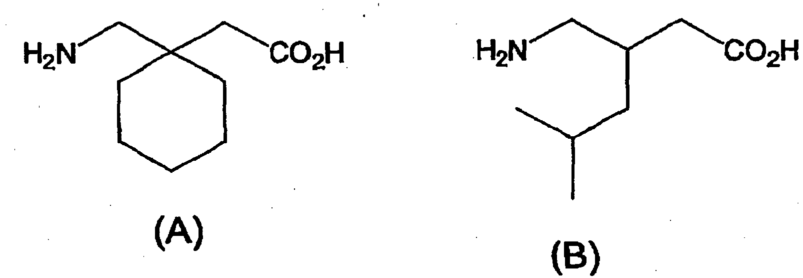

Aus dem Stand der Technik sind eine Reihe von Verfahren zur Herstellung von Gabapentin bzw. Pregabalin bekannt Beispielhaft wird auf ein Verfahren von J.S. Bryans et al. (J. Med. Chem. 41, 1838 -1845 (1998)) zur Herstellung von Gabapentin verwiesen.

Entsprechend diesem Verfahren können aus Ketonen oder Aldehyden durch Wadsworth-Emmons-Olefinierung mit Triethylphosphonoacetat, in Gegenwart von Natriumhydrid als Base und in Tetrahydrofuran als Lösungsmittel Acrylsäureethylester hergestellt werden. Anschließend wird über eine Michael-Additon Nitromethan addiert Die Nitrogruppe wird zur Aminogruppe reduziert, wobei die gebildeten Verbindungen zu γ-Lactamen cyclisieren. Durch saure Hydrolyse können die γ-Aminosäuren gewonnen werden.

Nachteilig an diesem bekannten Verfahren ist die Verwendung von Alkalihydriden zur Herstellung von substituierten Acrylsäureestern in der ersten Reaktionsstufe, wodurch die Verwendung von absoluten organischen Lösungsmitteln und das Arbeiten unter Schutzgas erforderlich ist.

Villieras et al. (Synthesis 1983, Seiten 300 bis 303) beschreiben die Synthese von α,β-ungesättigten Estern durch Wadsworth-Emmons-Olefinierung in heterogenen Medien.

Vasil'ev et al. (Bioorganic & Medicinal Chemistry 1996, Seiten 389 bis 400) beschreiben die Synthese von Insektenpheromonen durch Horner-Emmons-Synthese.

Barbot et al. (Journal of Organometallic Chemistry 1988, Seiten 239 bis 243) offenbaren die Reaktion von Lithiumorganocupraten mit ungesättigten Estern, die über eine Wadsworth-Emmons-Olefinierung erhalten wurden.

Gomez-Monterrey et al. (Journal of Medicinal Chemistry 1993, Seiten 87 bis 94) offenbaren die Synthese von Inhibitoren, die Thiolgruppen umfassen.

Aufgabe der vorliegenden Erfindung war es daher, die erste Reaktionsstufe zur Herstellung von substituierten Acrylsäureestern so zu verbessern, daß ohne absolute organische Lösungsmittel und ohne Schutzgas gearbeitet werden kann und die substituierten Acrylsäureester in hoher Reinheit und Ausbeute erhalten werden, wodurch auch das vorstehend beschriebene Verfahren zur Herstellung von substituierten γ-Aminosäuren, wie Gabapentin und Pregabalin, vereinfacht wird.

Erfindungsgemäß wird die Aufgabe durch Bereitstellung der nachstehend beschriebenen Verfahren zur Herstellung von substituierten Acrylsäureestern und daraus hergestellten substituierten γ-Aminosäuren, wie Gabapentin und Pregabalin, gelöst.

Gegenstand der vorliegenden Erfindung ist daher ein Verfahren zur Herstellung von substituierten Acrylsäureestern der allgemeinen Formel II, 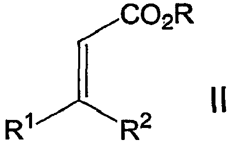 worin
R für einen linearen oder verzweigten, gesättigten oder ungesättigten aliphatischen C₁₋₃-Rest, vorzugsweise eine Ethylgruppe, steht,
R¹ für Wasserstoff oder einen linearen oder verzweigten, gesättigten oder ungesättigten aliphatischen C₁₋₆-Rest, vorzugsweise einen C₁₋₃-Rest, steht und
R² für einen linearen oder verzweigten, gesättigten aliphatischen C₁₋₆-Rest, vorzugsweise einen C₁₋₄-Rest, steht oder
R¹ und R² zusammen eine gesättigte Kohlenwasserstoff-Kette unter Ausbildung eines 3-8-gliedrigen cycloaliphatischen Ringes bilden,
in dem man
ein Keton oder Aldehyd der allgemeinen Formel V, worin R¹ und R² die vorstehend genannte Bedeutung haben, 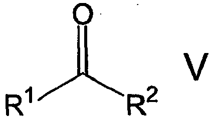 mit einem Trialkylphophonoacetat der allgemeinen Formel VI, worin R für einen linearen oder verzweigten, gesättigten oder ungesättigten aliphatischen C₁₋₃-Rest, vorzugsweise eine Ethylgruppe, steht, 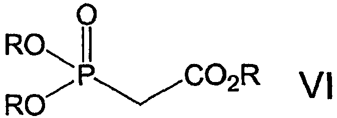 in Gegenwart von Alkalicarbonat, vorzugsweise Kaliumcarbonat, in einem wäßrigem Lösungsmittel, vorzugsweise Wasser, umsetzt.

Erfindungsgemäß werden die Verbindungen der allgemeinen Formeln V und VI sowie das Alkalicarbonat, vorzugsweise das Kaliumcarbonat, bei einer Temperatur im Bereich von 0 bis 10°C, vorzugsweise unter Eiskühlung, vereinigt und bei einer Temperatur im Bereich von ≤ 25 °C, vorzugsweise unter Eiskühlung, zu einem substituierten Acrylsäureester der allgemeinen Formel II umgesetzt. Vorzugsweise wird die Temperatur durch Eiskühlung vor und während der Umsetzung reguliert.

Im Anschluß an die Umsetzung kann der substituierte Acrylsäureester der allgemeinen Formel II, vorzugsweise durch Extraktion, gereinigt werden, wobei Diethylether als Lösungsmittel bevorzugt ist.

Besonders bevorzugt ist ein Verfahren, in dem man Cyclohexanon bzw. 3-Methylbutanal als Verbindung der allgemeinen Formel V mit Triethylphosphonoacetat als Verbindung der allgemeinen Formel VI jeweils zu Cyclohexylidenessigsäureethylester bzw. **5-Methylhex-2-ensäureethylester** umsetzt.

Substituierte Acrylsäureester der allgemeinen Formel II werden nach dem erfindungsgemäßen Verfahren in hoher Ausbeute und Reinheit gewonnen. Die Olefnierungen sind sowohl im 0,5-molaren Maßstab, wie im mehr-molaren Maßstab ohne Verschlechterung der Reinheit oder der Ausbeute durchführbar. Die erfindungsgemäß hergestellten substituierten Acrylsäureester der allgemeinen Formel II können nach Reinheitskontrolle durch GC/MS-Analytik sofort weiter umgesetzt werden. Im weiteren Syntheseverlauf zur Herstellung von substituierten γ-Aminosäuren können die vorstehend aufgeführten Syntheseschritte ohne Änderungen durchgeführt werden. Für die entsprechende Herstellung wird die Offenbarung von J.S. Bryans et al. (J. Med. Chem. 41, 1838 - 1845 (1998)) hiermit als Referenz eingeführt.

Ein weiterer Gegenstand der vorliegenden Erfindung sind daher Verfahren zur Herstellung einer substituierten γ-Aminosäure der allgemeinen Formel I, worin R¹ und R² die vorstehend genannte Bedeutung haben, 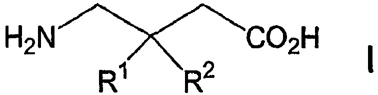 in denen man
a) einen substituierten Acrylsäureester der allgemeinen Formel **II** nach dem vorstehend beschriebenen erfindungsgemäßen Verfahren herstellt, 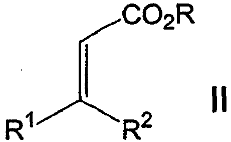 unter Schutzgasatmosphäre unter Addition von Nitromethan zu einer Verbindung der allgemeinen Formel III umsetzt, vorzugsweise in Gegenwart von Tetrabutylammoniumfluorid als Katalysator in einem geeigneten Lösungsmittel, vorzugsweise Tetrahydrofuran, 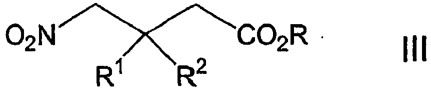
b) die Verbindung der allgemeinen Formel III zu einem Lactam der Formel IV reduziert, vorzugsweise durch Umsetzung mit Wasserstoff in Gegenwart eines geeigneten Katalysators, vorzugsweise Raney-Nickel, in einem geeigneten Lösungsmittel, vorzugsweise Methanol, 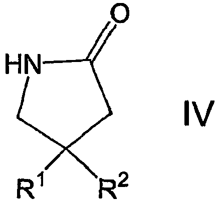
c) das Lactam der allgemeinen Formel IV zu einer γ-Aminosäure der Formel I öffnet, vorzugsweise durch Umsetzung mit einer Säure, vorzugsweise Salzsäure, in einem geeigneten Lösungsmittel, vorzugsweise Dioxan. Besonders bevorzugt ist ein Verfahren, in dem man Cyclohexanon bzw. 3-Methylbutanal als Verbindung der allgemeinen Formel V mit Triethylphosphonoacetat als Verbindung der allgemeinen Formel VI jeweils zu Cyclohexylidenessigsäureethylester bzw. **5-Methylhex-2-ensäureethylester** in der ersten Reaktionsstufe umsetzt und wie angegeben weiter zu Gabapentin bzw. Pregabalin verarbeitet.
   In dem erfindungsgemäßen Verfahren zur Herstellung von substituierten Acrylsäureestern bzw. bei der ersten Reaktionsstufe zur Herstellung von substituierten γ-Aminosäuren, wie Gabapentin und Pregabalin, werden Alkalicarbonate als Base eingesetzt. Daher kann bei der Wadsworth-Emmons-Olefinierung nach den erfindungsgemäßen Verfahren auf Schutzgas und absolute organische Lösungsmittel verzichtet und wäßrige Lösungsmittel, vorzugsweise Wasser, verwendet werden.

Substituierte γ-Aminosäuren, wie Gabapentin und Pregabalin werden als Arzneimittel zur Behandlung von Epilepsie und Schmerzzuständen verwendet.

Im folgenden wird die Erfindung anhand von Beispielen erläutert. Diese Erläuterungen sind lediglich beispielhaft und schränken den allgemeinen Erfindungsgedanken nicht ein.

### Beispiele

Die eingesetzten Chemikalien und Lösungsmittel wurden kommerziell bei den herkömmlichen Anbietern erworben (Acros, Aldrich, Fluka, Lancaster und Merck).

Die NMR-Spektren wurden mit Spektrometem der Firma Bruker Analytik GmbH, Silberstreifen 4, D-76287 Rheinstetten gemessen. Die Gerätebezeichnungen lauten: für 300 MHz: Avance DPX 300 MHz, für 600 MHz: Avance DRX 600 MHz.

Die GC-Analytik wurde an einem Gaschromatographen HP 6890 (mit PTV-Injektor) und einem gekoppelten Massen-Detektor 5973 (Mass selective Detector) der Firma Hewlett-Packard durchgeführt.

Die ESI-Massenspektren wurden mit einem Gerät vom Typ Finnigan LCQ der Firma Thermoquest, Analytische Systeme GmbH, Boschring 12, D-63329 Egelsbach gemessen und mit der Software Xcalibur ausgewertet.

### Beispiel 1

### Synthese von Gabapentin-Hydrochlorid

### 1.1. Synthese von Cyclohexylildenesalgsäureethylester

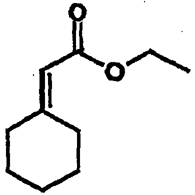

31,1 ml (0,3 mol) Cyclohexanon, 72,1 ml (0,36 mol) Triethylphosphonoacetat und 83 g (0,6 mol) Kaliumcarbonat wurden in 60 ml Wasser unter Eiskühlung zusammengegeben und 20 Stunden unter langsamem Erwärmen auf 25°C gerührt.

Der Reaktionsansatz wurde mit 50 ml Wasser verdünnt und dreimal mit Ether extrahiert. Die organische Phase wurde mit Wasser neutral gewaschen, über MgSO₄ getrocknet und eingeengt. Cyclohexylidenessigsäuroethylester wurde als leicht gelbliche Flüssigkeit in einer Ausbeute von 47,4 g (94% der Theorie) gewonnen.

MS (berechnet: 168,34 g/mol): 168 (M⁺), 153 (M⁺-CH₃), 139 (M⁺-CH₂CH₃), 123 (M⁺-OCH₂CH₃), 95 (M⁺-CO₂CH₂CH₃).

¹H-NMR (CDCl₃/TMS_{int.}): δ = 1,27 ppm (3H, t, CH₃, J = 7,2 Hz); 1,64 ppm (6H, m, CH₂); 2,19 ppm (2H, m, CH₂); 2,83 ppm (2H, m, CH₂); 4,14 ppm (2H, q, OCH₂, J = 7,1 Hz); 5,60 ppm (1 H, s, Olefin-H).

### 1.2. Synthese von (1-Nitromethyl-cyclohexyl)-essigsäureethylester

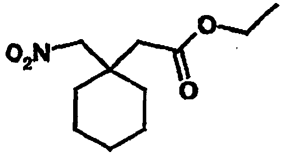

In 50 ml Tetrahydrofuran wurden 14,8 g (0,088 mol) des nach 1.1. hergestellten Cyclohexylidenessigsäureethylester gelöst, unter Stickstoffatmosphäre 7,11 ml (0,132 mol) Nitromethan und 88 ml Tetrabutylammoniumfluorid (1-molar in Tetrahydrofuran) zugegeben und 20 Stunden unter Rückfluß gekocht. Nach dem Abkühlen wurde mit 50 ml Wasser verdünnt und dreimal mit Diethylether extrahiert. Die organische Phase wurde mit 10 Gew.-%-iger wäßriger Kaliumhydrogensulfat-Lösung und anschließend mit Wasser gewaschen, über Magnesiumsulfat getrocknet und eingeengt (1-Nitromethyl-cyclohexyl)-essigsaureothylester wurde als orangegelbe, ölige Flüssigkeit erhalten. Die Ausbeute betrug 19,8 g (98% der Theorie)

MS (berechnet 229,28 g/mol): 230 (M⁺), 184 (M⁺-OCH₂CH₃), 169 (M⁺-CH₂NO₂).

¹H-NMR (CDCl₃/TMS_{int.}): δ = 1,27 ppm (3H, t, CH₃, J = 7.2 Hz); 1,48 - 1,53 ppm (10, m, CH₂); 2,54 ppm (2H, s, CH₂); 4,15 ppm (2H, q, OCH₂, J = 7,2 Hz); 4,71 ppm (2H, s, CH₂).

### 1.3. Synthese von 2-Azaspiro[4.5]decan-3-on

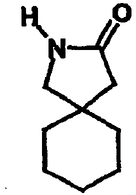

22 g (0,096 mol) eines gemäß 1.2. hergestellten (1-Nitromethyl-cyclohexyl)-essigsäureethylesters wurden in 150 ml Methanol gelöst, 2,20 g Raney-Nickel zugegeben und 20 Stunden unter kontinuierlicher Zufuhr von Wasserstoff hydriert. Der Reaktionsansatz wurde über Filtererde abfiltriert, mit Methanol nachgewaschen und am Rotationsverdampfer eingeengt. Es wurden 15,3 g (100% Ausbeute) einer gelblichen Kristallmasse erhalten.
Durch Verrühren mit Hexan und Abfiltrieren wurden 12,4 g (81 %Ausbeute) farblose kristalle erhalten.
2-Azaspiro[4.5]decan-3-on wurde als Rohprodukt in der weiteren Synthese eingesetzt.

MS (berechnet: 153,22 g/mol): 153 (M⁺), 125 (M⁺-CO), 123 (M⁺-CH₂NH), 96 (M⁺-CH₂NHCO), 81 (M⁺-CH₂NHCOCH₃).

¹H-NMR (CDCl₃/TMS_{int.}): δ = 1,43 ppm (10H, m, CH₂); 1,98 ppm (2H, s, CH₂); 2,99 ppm (2H, s, CH₂); 7,20 ppm (1H, s (breit), NH).

### 1.4. Synthese von (1-Aminomethyl-cyclohexyl)-essigsäure-Hydrochlorid (Gabepentin-Hydrochlorid)

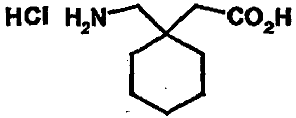

12,4 g (0,81 mol) des nach 1.3. hergestellten 2-Azaspiro[4.5]decan-3-on wurden in 4-nomaler Salzsäure aufgenommen und 20 Stunden bei 110°C Badtemperatur gerührt. Nach dem Abkühlen wurde mit Wasser verdünnt und zweimal mit Dichlormethan extrahiert. Die wäßrige Phase wurde mit Aktivkohle verrührt, über Filtererde abfiltriert, eingeengt und im Hochvakuum getrocknet. Der farblose Feststoff wurde in Methanol/Aceton (5/3, v/v) gelöst und durch Zugabe von Diethylether gefällt. Nach Abfiltrieren, Waschen mit Diethylether wurden 12,4 g (74% der Theorie) von (1-Aminomethyl-cyclohexyl)-essigsäure-Hydrochlorid erhalten.

ESI-MS (berechnet: 168,34 g/mol):

¹H-NMR (d₆-DMSO/TMS_{ext.}): δ = 1,40 ppm (10H, m, 5 x CH₂); 2,40 ppm (2H, m, CH₂); 2,90 ppm (2H, m, CH₂); 8,00 ppm (3H, s (breit), NH₃⁺); 12,30 ppm (1H, s (breit), CO₂H).

### Beispiel 2

### Synthese von Pregabalin-Hydrochiorid

### 2.1. Synthese von (2E)-5-Methylhex-2-ensäureethylester

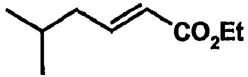

1 mol (138 g) Kaliumcarbonat wurde in 100 ml Wasser gelöst und in Eiswasser gekühlt. Es wurden 0,6 mol (120 ml) Phosphonoessigsäuretrieethylester und 0,5 mol (43,1 g) 3-Methylbutanal zugegeben und 20 Stunden unter langsamer Erwärmung auf 25°C gerührt. Anschließend wurde der Reaktionsansatz mit 150 ml Wasser verdünnt und viermal mit Diethylether extrahiert. Die organische Phase wurde mit Magnesiumsulfat getrocknet und am Rotationsverdampfer eingeengt. Es wurde eine klare Flüssigkeit erhalten.

Die Rohausbeute betrug 75,8 g (97% der Theorie).

Die gaschromatographische Untersuchung zeigte, daß das Rohprodukt nur Spuren der beiden Edukte enthielt. Es wurde ohne weitere Reinigungsschritte in die nächste Synthesestufe eingesetzt.

MS (berechnet: 156,23 g/mol): 156 (M⁺), 141 (M⁺-CH₂), 128 (M⁺-CH₂CH₃), 111 (M⁺-OCH₂CH₃), 86 (M⁺-(CH₃)₂CHCH₂).

¹H-NMR (d₆-DMSO/TMS_{ext.}): δ = 0,89 ppm (6H, d, 2 x CH₃, J = 6,8 Hz); 1,22 ppm (3H, t, CH₃, J = 7,2 Hz); 1,76 ppm (1H, h, C*H*(CH₃)₂, J = 6,5 Hz, J = 6,8 Hz); 2,01 ppm (2H, m, CH₂, J = 6,8 Hz); 4,11 ppm (2H, q, CH₂O, J = 7,2 Hz); 5,83 ppm (1 H, dd, CH, J = 1,5 Hz, J = 15,5 Hz); 6,85 ppm (1 H, m, CH), J = 7,5 Hz).

### 2.2. Synthese von 5-Methyl-3-nitromethyl-hexansäureethylester

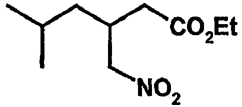

0,485 mol (75,8 g) des nach 2.1. hergestellten (2*E*)-5-Methyl-hex-2-en-**säureethylester** wurden unter Stickstoffatmosphäre in THF (abs.) gelöst. 0,73 mol (39,1 ml) Nitromethan und 0,49 mol (485 ml) einer 1-molaren Tetrabutylammoniumfluorid-Lösung in THF wurden zugegeben, wobei eine orange Lösung entstand. Es wurde 20 Stunden unter Rückfluß gekocht. Nach dem Abkühlen wurde mit 280 ml Wasser versetzt und viermal mit Diethylether extrahiert. Die organische Phase wurde dreimal mit 10%-iger wäßriger KaliumhydrogensulfatLösung extrahiert und mit Wasser neutral gewaschen. Die organische Phase wurde mit Magnesiumsulfat getrocknet und am Rotationsverdampfer eingeengt. Es wurde eine orange Flüssigkeit erhalten.
Die Rohausbeute betrug 95,2 g (90% der Theorie).

Die gaschromatographische Untersuchung zeigte, daß im Rohprodukt neben 91 % Produkt noch 7% Tetrabutylammoniumfluorid und 2% nicht identifizierbares Nebenprodukt enthalten waren.
Zur Reinigung wurde das Rohprodukt 4 Stunden in Ether gerührt, die Lösung filtiert und eingeengt.

MS (berechnet: 217,27 g/mol): 202 (M-CH₃), 172 (M-OCH₂CH₃), 156 (M-CH₂NO₂), 143 (M-CO₂CH₂CH₃).

¹H-NMR (d₆-DMSO/TMS_{ext.}): δ = 0,96 ppm (6H, t, 2 x CH₃, J = 6,8 Hz); 1,14 - 1,21 ppm (5H, m, CH₂ und CH₃, J = 7,2 Hz); 1,63 ppm (1H, sept., CH, J = 6,8 Hz); 2,38 ppm (2H, d, CH₂CO₂Et, J = 6,4 Hz); 2,58 ppm (1 H, sept., CH, J = 6,4 Hz, J = 6,8 Hz); 4,07 ppm (2H, q, OCH₂, J = 7,0 Hz); 4,55 ppm (2H, d, CH₂NO₂, J = 6,4 Hz).

### 2.3. Synthese von 4-Isobutyl-pyrrolidin-2-on

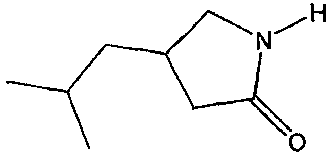

0,39 mol (85,3 g) des nach 2.2. hergestellten und gereinigten 5-Methyl-3-nitromethyl-**hexansäureethylester** wurden in ca. 900 ml Methanol gelöst, 25 g Raney-Nickel zugegeben und bei einer Temperatur von 35°C und einem Druck von 2 bar 20 Stunden hydriert. Der Reaktionsansatz wurde über Filtererde abfiltriert, vorsichtig mit Methanol gewaschen und am Rotationsverdampfer eingeengt. Nach Trocknen im Hochvakuum wurden 59,3 g einer farblosen Flüssigkeit erhalten.

MS (berechnet: 141,21 g/mol): 141 (M⁺), 126 (M⁺-CH₃), 111 (M⁺-CO), 98 (M⁺-CH(CH₃)₂), 84 (M⁺-CH₂NHCOCH₃).

¹H-NMR (d₆-DMSO/TMS_{ext.}): δ = 0,87 ppm (3H, d, CH₃, J = 6,4 Hz); 0,88 ppm (3H, d, CH₃, J = 6,4 Hz); 1,28 ppm (2H, t, CH₂, J = 7,2 Hz); 1,55 ppm (1H, sept, CH, J = 6,4 Hz, 8,8 Hz); 1,79 ppm (1H, dd, CH, J = 8,7 Hz); 2,20 ppm (1H, dd, CH, J = 8,3 Hz); 2,40 ppm (1H, sept, CH, J = 7,5 Hz, J = 8,3 Hz); 2,83 ppm (1H, dd, CH, J = 7,5 Hz); 3,33 ppm (1H, dd, CH, J = 8,3 Hz), 7,46 ppm (s (breit), 1H, NH).

### 2.4. Synthese von 3-Aminomethyl-5-methyl-hexansäure-Hydrochlorid (Pregabalin-Hydrochlorid)

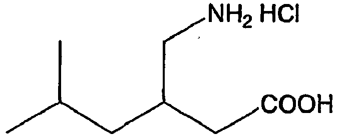

7,16 g (0,06 mol) des nach 2.3. hergestellten 4-Isobutyl-pyrrolidin-2-on wurden in 550 ml 4-normaler Salzsäure aufgenommen und 20 Stunden bei einer Ölbadtemperatur von 125°C erhitzt. Nach dem Abkühlen wurde mit 500 ml Wasser verdünnt und dreimal mit Dichlormethan extrahiert. Die wäßrige Phase wurde mit Aktivkohle verrührt, über Filterende abfiltriert und eingeengt. Nach Trocknen im Hochvakuum wurde das 3-Aminomethyl-5-methyl-hexansäure-Hydrochlorid als orange-gelbe Kristallmasse in einer Ausbeute von 100% erhalten.

ESI-MS (berechnet: 159,23 g/mol): 160 (MH⁺). 142 (MH+-H2O).

¹H-NMR (d₆-DMSO/TMS_{ext.}): 8 = 0,88 ppm (6H, dd, 2 x CH₃, J = Hz); 1,18 ppm (1H, dd, CH₂, J = Hz); 1,20 ppm (1H, dd, CH₂, J = Hz); 1,61 ppm (1H, sept, CH, J = Hz); 2,18 ppm (2H, m, CH₂, J = Hz); 2,45 ppm (1H, dd, CH, J = Hz); 2,78 ppm (2H, m, CH₂, J = Hz); 8,10 ppm (3H. s (breit), NH₃⁺).

## Patentansprüche

1. Verfahren zur Herstellung substituierter Acrylsäureester der allgemeinen Formel II, 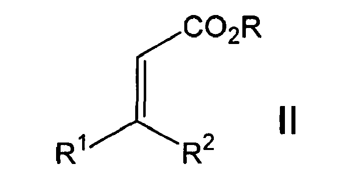 worin R für einen linearen oder verzweigten, gesättigten oder ungesättigten aliphatischen C₁₋₃-Rest steht,
R¹ für Wasserstoff oder einen linearen oder verzweigten, gesättigten oder ungesättigten aliphatischen C₁₋₆-Rest steht und
R² für einen linearen oder verzweigten, gesättigten aliphatischen C₁₋₆-Rest steht, oder R¹ und R² zusammen eine gesättigte Kohlenwasserstoff-Kette unter Ausbildung eines 3-8-gliedrigen cycloaliphatischen Ringes bilden,
**dadurch gekennzeichnet, daß** man
ein Keton oder einen Aldehyd der allgemeinen Formel V, worin R¹ und R² die vorstehend genannte Bedeutung haben, 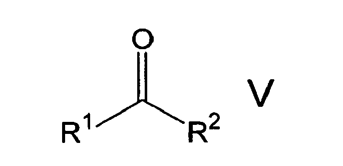 mit Trialkylphosphonoacetat der Formel VI, worin R für einen linearen oder verzweigten, gesättigten oder ungesättigten aliphatischen C₁₋₃-Rest steht, 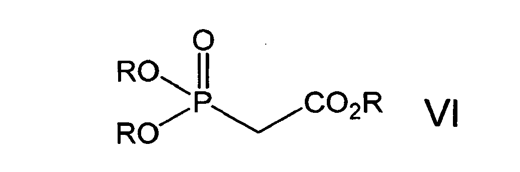 und Alkalicarbonat als Base in einem wäßrigen Lösungsmittel bei einer Temperatur im Bereich von 0 bis 10°C vereinigt und bei einer Temperatur im Bereich von ≤ 25°C umsetzt.

2. Verfahren zur Herstellung substituierter Acrylsäureester der allgemeinen Formel II gemäß Anspruch 1, worin
R für einen linearen oder verzweigten, gesättigten oder ungesättigten aliphatischen C₁₋₃-Rest steht,
R¹ für Wasserstoff oder einen linearen oder verzweigten, gesättigten oder ungesättigten aliphatischen C₁₋₃-Rest steht und
R² für einen linearen oder verzweigten, gesättigten aliphatischen C₁₋₄-Rest steht, oder R¹ und R² zusammen eine gesättigte Kohlenwasserstoff-Kette unter Ausbildung eines 3-8-gliedrigen cycloaliphatischen Ringes bilden.

3. Verfahren gemäß Anspruch 1 oder 2, **dadurch gekennzeichnet, daß** man ein Keton oder einen Aldehyd der allgemeinen Formel V mit Trialkylphosphonoacetat der allgemeinen Formel VI, worin R für eine Ethylgruppe steht, in Gegenwart von Alkalicarbonat, vorzugsweise Kaliumcarbonat, in Wasser umsetzt.

4. Verfahren gemäß einem der Ansprüche 1-3, **dadurch gekennzeichnet, daß** die Vereinigung und/oder die Umsetzung, vorzugsweise die Vereinigung und die Umsetzung, unter Eiskühlung erfolgen.

5. Verfahren gemäß einem der Ansprüche 1-4, **dadurch gekennzeichnet, daß** man den substituierten Acrylsäureester der allgemeinen Formel II im Anschluß an die Umsetzung durch Extraktion reinigt.

6. Verfahren gemäß Anspruch 5, **dadurch gekennzeichnet, daß** man mit Diethylether extrahiert.

7. Verfahren gemäß einem der Ansprüche 1-6, **dadurch gekennzeichnet, daß** man Cyclohexanon bzw. 3-Methylbutanal als Verbindung der allgemeinen Formel V mit Triethylphosphonoacetat als Verbindung der allgemeinen Formel VI jeweils zu Cyclohexylidenessigsäureethylester bzw. 5-Methylhex-2-ensäureethylester umsetzt.

8. Verfahren zur Herstellung einer substituierten γ-Aminosäure der allgemeinen Formel I, worin R¹ und R² die in Anspruch 1 oder 2 genannte Bedeutung haben, 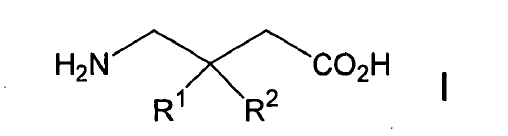 in dem man
b) einen substituierten Acrylsäureester der allgemeinen Formel II, worin R, R¹ und R² die in Anspruch 1, 2 oder/und 3 genannte Bedeutung haben, 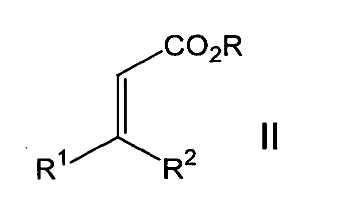 unter Schutzgasatmosphäre unter Addition von Nitromethan in bekannter Weise zu einer Verbindung der allgemeinen Formel III umsetzt, 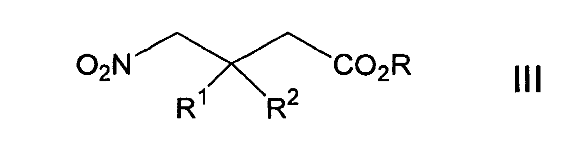
c) die Verbindung der allgemeinen Formel III in bekannter Weise zu einem Lactam der allgemeinen Formel IV reduziert und 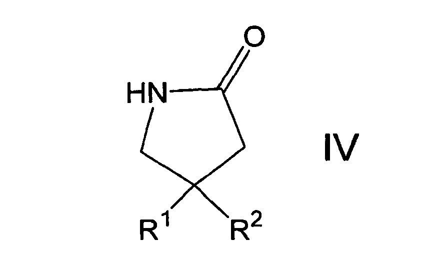
d) das Lactam der allgemeinen Formel IV in bekannter Weise mit Hilfe von Säure unter Bildung einer substituierten γ-Aminosäure der allgemeinen Formel I spaltet,
**dadurch gekennzeichnet, daß** man
a) den substituierten Acrylsäureester der allgemeinen Formel II nach einem Verfahren gemäß einem der Ansprüche 1-7 herstellt.

9. Verfahren nach Anspruch 8, **dadurch gekennzeichnet, daß** man Cyclohexylidenessigsäureethylester bzw. 5-Methylhex-2-ensäureethylester nach Anspruch 7 herstellt und zu Gabapentin bzw. Pregabalin umsetzt.

## Claims

1. A process for the production of substituted acrylic
acid esters of the general formula II, 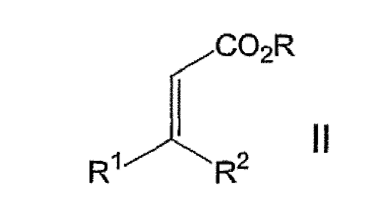 in which R denotes a linear or branched, saturated or unsaturated aliphatic C₁₋₃ residue,
R¹ denotes hydrogen or a linear or branched, saturated or unsaturated aliphatic C₁₋₆ residue and
R² denotes a linear or branched, saturated or unsaturated aliphatic C₁₋₆ residue, or
R¹ and R² together form a saturated hydrocarbon chain with the formation of a 3-8-membered cycloaliphatic ring,
**characterised in that**
a ketone or an aldehyde of the general formula V, in which R¹ and R² have the above-stated meaning, 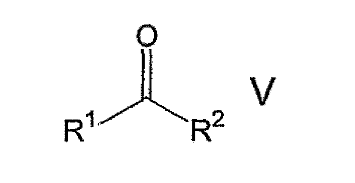 is combined with trialkyl phosphonoacetate of the formula VI, in which R denotes a linear or branched, saturated or unsaturated aliphatic C₁₋₃ residue, 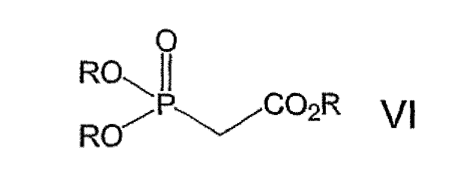 and alkali metal carbonate as base in an aqueous solvent at a temperature in the range from 0 to 10°C and reacted at a temperature in the range of ≤ 25°C.

2. A process for the production of substituted acrylic acid esters of the general formula II, according to claim 1, in which
R denotes a linear or branched, saturated or unsaturated aliphatic C₁₋₃ residue,
R¹ denotes hydrogen or a linear or branched, saturated or unsaturated aliphatic C₁₋₃ residue and
R² denotes a linear or branched, saturated or unsaturated aliphatic C₁₋₄ residue, or
R¹ and R² together form a saturated hydrocarbon chain with the formation of a 3-8-membered cycloaliphatic ring.

3. A process according to claim 1 or 2, **characterised in that** a ketone or an aldehyde of the general formula V is reacted with trialkyl phosphonoacetate of the general formula VI, in which R denotes an ethyl group, in the presence of alkali metal carbonate, preferably potassium carbonate, in water.

4. A process according to any one of claims 1-3, **characterised in that** the combination and/or the reaction, preferably the combination and the reaction, proceed with ice cooling.

5. A process according to any one of claims 1-4, **characterised in that**, after the reaction, the substituted acrylic acid ester of the general formula II is purified by extraction.

6. A process according to claim 5, **characterised in that** extraction is performed with diethyl ether.

7. A process according to any one of claims 1-6, **characterised in that** cyclohexanone or 3-methylbutanal as a compound of the general formula V is reacted with triethyl phosphonoacetate as a compound of the general formula VI in each case to yield cyclohexylideneacetic acid ethyl ester or 5-methylhex-2-enoic acid ethyl ester respectively.

8. A process for the production of a substituted γ-amino acid of the general formula I, in which R¹ and R² have the meaning stated in claim 1 or 2, 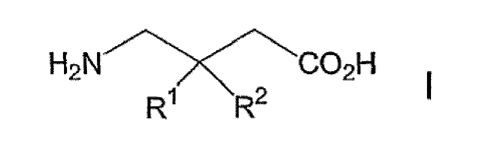 in which
b) a substituted acrylic acid ester of the general formula II, in which R, R¹ and R² have the meaning stated in claim 1, 2 and/or 3, 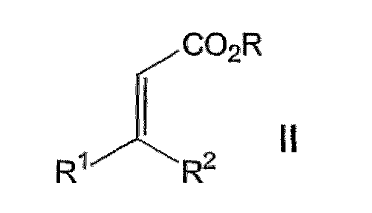 is reacted under a protective gas atmosphere with the addition of nitromethane in known manner to yield a compound of the general formula III, 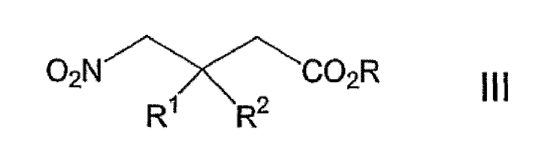
c) the compound of the general formula III is reduced in known manner to yield a lactam of the general formula IV and 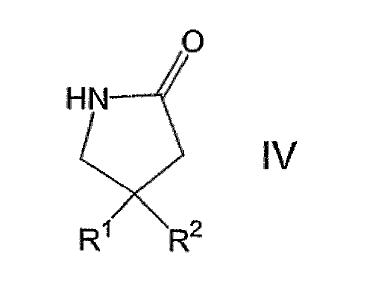
d) the lactam of the general formula IV is cleaved in known manner with the assistance of acid with formation of a substituted γ-amino acid of the general formula I,
**characterised in that**
a) the substituted acrylic acid ester of the general formula II is produced using a process according to any one of claims 1-7.

9. A process according to claim 8, **characterised in that** cyclohexylideneacetic acid ethyl ester or 5-methylhex-2-enoic acid ethyl ester is produced according to claim 7 and reacted to yield gabapentin or pregabalin respectively.

## Revendications

1. Procédé pour la préparation d'esters d'acide acrylique substitués de formule générale II, 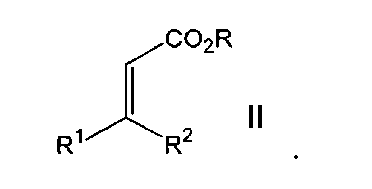 dans laquelle R représente un radical aliphatique en C₁-C₃ linéaire ou ramifié, saturé ou insaturé,
R¹ représente un atome d'hydrogène ou un radical aliphatique en C₁-C₆ linéaire ou ramifié, saturé ou insaturé et
R² représente un radical aliphatique en C₁-C₆ linéaire ou ramifié, saturé ou
R¹ et R² forment ensemble une chaîne hydrocarbonée saturée, avec formation d'un cycle cycloaliphatique à 3-8 chaînons,
**caractérisé en ce**
**qu'**on réunit une cétone ou un aldéhyde de formule générale V, dans laquelle R¹ et R² ont la signification indiquée précédemment, 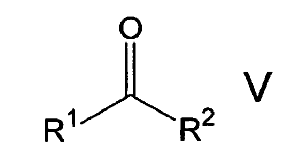 avec un phosphonoacétate de trialkyle de formule VI, dans laquelle R représente un radical aliphatique en C₁-C₃ linéaire ou ramifié, saturé ou insaturé, 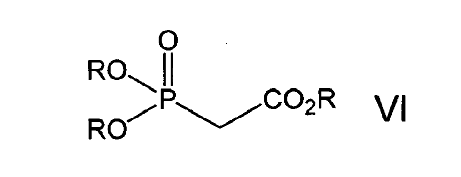 et un carbonate de métal alcalin en tant que base, dans un solvant aqueux, à une température dans la plage de 0 à 10 °C, et on les fait réagir à une température dans la plage de ≤ 25 °C.

2. Procédé pour la préparation d'esters d'acide acrylique substitués de formule générale II selon la revendication 1, dans lesquels
R représente un radical aliphatique en C₁-C₃ linéaire ou ramifié, saturé ou insaturé,
R¹ représente un atome d'hydrogène ou un radical aliphatique en C₁-C₃ linéaire ou ramifié, saturé ou insaturé et
R² représente un radical aliphatique en C₁-C₄ linéaire ou ramifié, saturé ou
R¹ et R² forment ensemble une chaîne hydrocarbonée saturée, avec formation d'un cycle cycloaliphatique à 3-8 chaînons.

3. Procédé selon la revendication 1 ou 2, **caractérisé en ce**
**qu'**on fait réagir une cétone ou un aldéhyde de formule générale V avec un phosphonoacétate de trialkyle de formule générale VI, dans laquelle R représente un groupe éthyle, en présence d'un carbonate de métal alcalin, de préférence de carbonate de potassium, dans de l'eau.

4. Procédé selon l'une quelconque des revendications 1 à 3, **caractérisé en ce que** la réunion et/ou la réaction, de préférence la réunion et la réaction, s'effectuent avec refroidissement avec de la glace.

5. Procédé selon l'une quelconque des revendications 1 à 4, **caractérisé en ce qu'**à la suite de la réaction on purifie par extraction l'ester d'acide acrylique substitué de formule générale II.

6. Procédé selon la revendication 5, **caractérisé en ce qu'**on effectue l'extraction avec de l'oxyde d'éthyle.

7. Procédé selon l'une quelconque des revendications 1 à 6, **caractérisé en ce qu'**on fait réagir de la cyclohexanone ou du 3-méthylbutanal en tant que composé de formule générale V, avec du phosphonoacétate de triéthyle en tant que composé de formule générale VI, pour obtenir du cyclohexylidène-acétate d'éthyle ou, respectivement, du 5-méthylhex-2-énoate d'éthyle.

8. Procédé pour la préparation d'un acide γ-aminé substitué de formule générale I, dans laquelle R¹ et R² ont la signification indiquée dans la revendication 1 ou 2, 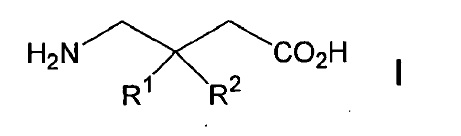 dans lequel
b) on fait réagir un ester d'acide acrylique substitué de formule générale II, dans laquelle R, R¹ et R² ont la signification indiquée dans la revendication 1, la revendication 2 ou/et la revendication 3, 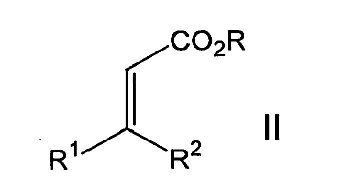 sous une atmosphère consistant en un gaz protecteur, avec addition de nitrométhane, d'une façon connue, pour aboutir à un composé de formule générale III, 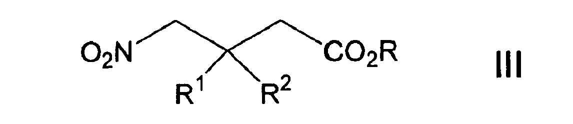
c) on réduit d'une façon connue le composé de formule générale III en un lactame de formule générale IV et 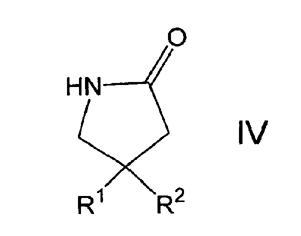
d) on coupe d'une façon connue le lactame de formule générale IV à l'aide d'un acide, avec formation d'un acide γ-aminé substitué de formule générale I ;
**caractérisé en ce que**
a) on prépare l'ester d'acide acrylique substitué de formule générale II conformément à un procédé selon l'une quelconque des revendications 1 à 7.

9. Procédé selon la revendication 8, **caractérisé en ce que** qu'on prépare selon la revendication 7 du cyclohexylidène-acétate d'éthyle ou du 5-méthylhex-2-énoate d'éthyle et on le convertit en gabapentine ou, respectivement, prégabaline.
